# EUROPEAN PATENT APPLICATION

(11) **EP 1 464 961 A1**
(43) Date of publication of application: **06.10.2004**
(21) Application number: 04251698.9
(22) Date of filing: 24.03.2004
(51) Int. Cl.: G01N 33/68, C12Q 1/68

(54) **METHODS AND REAGENTS FOR MULTIPLEXED ANALYSES OF PROTEINS**

(30) Priority: 02.04.2003 US 405438
(71) Applicant: Agilent Technologies, Inc., Palo Alto, CA 94306 (US)
(72) Inventor: Apffel, James Alexander, Mountain View California 94040 (US)
(74) Representative: Tollett, Ian

(57) **Abstract**

Methods, devices and kits are disclosed for detecting one or more target proteins in a sample suspected of containing a plurality of the target proteins. An assay medium comprising the sample is incubated with a surface (11a) of a substrate (11) comprising a plurality of capture agents (20, 22, 24) bound to the surface in a predetermined arrangement or pattern. For each target protein, at least two capture agents specific for different binding sites on the target protein are employed. The two capture agents are disposed on the surface of the substrate in a spatial relationship so that both bind to a molecule of the target protein, preferably the same molecule of the target protein. The binding to the molecule of the target protein is usually substantially simultaneous. The assay medium is incubated under conditions for binding of the capture agents to the target proteins. The substrate is then examined for the presence of the target proteins.

## Description

The present invention relates to methods, devices and kits for detecting one or more target proteins in a sample suspected of containing a plurality of such proteins. The invention has particular application to multiplexed protein analyses and to analyses utilizing polypeptide arrays.

Proteomics has come of interest over the last few years. While proteomics is more complex than genomics, the study of proteins gives more accurate pictures of cell biology than studying mRNA. The field of proteomics is very broad and involves areas such as, for example, protein profiling by the use of two-dimensional gel electrophoresis and mass spectrometry to study proteins expressed in the cell. Other areas include protein-protein interaction using yeast two-hybrid method and pathway analysis to understand signal transduction and other complex cell processes. Still other areas include large scale protein folding and 3-D structure studies and high-throughput expression and purification of proteins. Other areas include cellular expression during metabolism, mitosis, meiosis, in response to an external stimulus, e.g., drug, virus, change in physical or chemical condition, involving excess or deficient nutrients and cofactors, stress, aging, presence of particular strains of an organism and identifying the organism and strain, multiple drug resistance, protein-DNA interactions, protein-peptide interactions, and the like.

The human genome contains approximately 30,000 genes, of which 5,000-6,000 may be expressed in a given cell type. Although DNA sequencing of the human genome has been essentially completed, there remains the need to determine the functions of gene products. At the most basic level, genes code for proteins. In the past, the "central dogma" of modem biology states that a single gene codes for a single protein. However, in the last decade it has become clear that the real system is much more complex and in fact, a single gene may result in many different proteins due to alternate splicing and post-translational modifications such as glycosylation, phosphorylation, etc. Insights into gene function are provided by their expressed protein levels in different cell types, developmental stages, organism phenotypes, disease states, responses to stimuli, etc. Measuring these levels requires the initial resolution of complex mixtures of cellular proteins. Linkage of a specific gene to its protein product may then be established by sequencing or tryptic mapping of the protein and comparison with amino acid (AA) sequences predicted from DNA sequence databases.

There are a number of known methods for resolving cellular protein mixtures such as, for example, two-dimensional polyacrylamide gel electrophoresis (2D PAGE), which separates polypeptides based on the orthogonal parameters of isoelectric point (pI) and size. In an alternative technique, amino acid sequence data is obtained from single peptides by tandem mass spectrometry (MS/MS), and used to screen databases for unique protein sequences. In this technique, selected peptide masses are isolated in the first stage of the spectrometer and subjected to collision-induced chemical dissociation, and the masses of the subfragments are then analyzed in the second stage to deduce the AA sequence.

Because of the time and effort required for individual protein isolation, digestion and analysis, high-throughput strategies involving direct proteolysis and peptide analysis of protein mixtures have been demonstrated (Washburn, M.P., D. Wolters, and J.R. Yates, Large-scale analysis of the yeast proteome by multidimensional protein identification technology. Nature Biotechnology, 2001. 19(3): p. 242-247.). Such techniques include liquid chromatography (LC) coupled with MS/MS to separate and identify unique peptide sequences from tryptic digests of protein mixtures. Other techniques include MALDI (matrix assisted laser desorption ionization) or ESI (Electrospray Ionization) coupled with Ion Trap, Triple Quadrupole or QTOF Mass Spectrometry Analyzers. Alternatively, high resolution Fourier transform ion cyclotron resonance (FTICR)-MS may be employed to identify unique peptide masses in complex protein digests. All of the above approaches have one or more shortcomings.

Peptide and protein arrays on solid surfaces have received considerably less attention in comparison to DNA arrays. This is likely due to the inherent instability of these peptides and proteins at interfaces and in the presence of complex biological matrices as well as to the relatively complex interaction of target proteins and capture agents. For example, it is well known that many proteins denature upon contact with solid surfaces. Peptides, as well as proteins, are also subject to hydrolysis by any proteases that may be present in the biological sample being analyzed. Photolithographic techniques are commonly used (by Affymetrix, for example) for DNA arrays, but as yet not for protein arrays.

U.S. Patent Publication No. 20020055125 discloses an array of protein-binding agents stably attached to the surface of a solid support. The array includes a solid substrate having a substantially planar surface, and a plurality of different protein-binding agents bound to the substrate. Each of the protein-binding agents includes an anchoring segment stably bound to the substrate surface, a peptidomimetic protein-binding segment, and a linker segment connecting and separating the anchoring and peptidomimetic segments.

Rapid and high throughput quantitative multiplexed protein analysis is a rapidly developing technology. One class of approaches to this technology is based on the use of reagents (capture agents) that quantitatively and specifically capture target proteins (targets) and allow their detection and quantitation either through direct labeling or sandwich type assays. The samples can be presented to multiple such reagents in a range of formats including arrays, immobilized beads and physically separated wells in a microtiter plate. The capture agents themselves could be based on naturally occurring protein-binding moieties such as antibodies or can be engineered chemicals based on a range of scaffolds. In each case, the binding is presumed to occur between a specific site (epitope) on the target and the capture agent. The ability of the assay to quantitate one protein in the presence of other different proteins depends on the specificity of interaction between the target's epitope and the capture agent. If there is cross-reactivity or non-specific interaction between the capture agent and other proteins, the quantitative estimation of the target protein will be mis-estimated as higher than the true value.

There remains a need to perform analytical and diagnostic assays for proteins on a large scale. The assays should have high sensitivity and high specificity and permit multiplexed determinations. It is desirable to have a means for identifying a large number of proteins in a single sample, as well as providing some quantitation of the different proteins being detected.

One embodiment of the present invention is a method for detecting one or more target proteins in a sample suspected of containing a plurality of the target proteins. An assay medium comprising the sample is incubated with a surface of a substrate comprising a plurality of capture agents bound to the surface. The capture agents comprise for each target protein at least two capture agents specific for different binding sites on the target protein. The two capture agents are disposed on the surface of the substrate in a spatial relationship so that both bind to a molecule of the target protein, preferably the same molecule of the target protein. The binding to the molecule of the target protein is usually substantially simultaneous. The assay medium is incubated under conditions for binding of the capture agents to the target proteins. The substrate is then examined for the presence of said target proteins.

Another embodiment of the present invention is a method for determining one or more different target proteins in a sample suspected of containing a plurality of the target proteins. An assay medium comprising the sample is incubated with a surface of a substrate comprising an array of capture agents bound to the surface. At least two capture agents specific for different binding sites on each of the different target proteins are employed. The two capture agents both bind to the target protein, preferably the same molecule of target protein. The assay medium is incubated under conditions for binding of the capture agents to the target proteins. The substrate surface is then examined for the presence of the target proteins.

Another embodiment of the present invention is a device for determining one or more different target proteins in a sample suspected of containing a plurality of the target proteins. The apparatus comprises a solid substrate comprising a surface and an array of capture agents bound to the surface. At least two capture agents specific for different binding sites on each of the target proteins are present on the surface. The two capture agents are spatially disposed to both bind to a single molecule of the target protein.

Preferred embodiments of the present invention are described below, by way of example only, with reference to the accompanying drawings:
Fig. 1 is a perspective view of a substrate bearing multiple arrays.
Fig. 2 is an enlarged view of a portion of Fig. 1 showing some of the identifiable individual regions (or "features") of a single array of Fig. 1.
Fig. 3 is an enlarged cross-section of a portion of Fig. 2.

In a broad aspect, the present invention uses multiple independent surface-bound capture agents to analyze a complex mixture of different target proteins in a sample. For each target protein, at least two capture agents are employed, each of which binds to a different portion of a molecule of the target protein. The capture agent may be a polynucleotide, a peptide, a polypeptide, an antibody mimic, a display protein, a peptide mimetic, and the like. In one example, multiple pairs of capture agents are bound in a predetermined pattern or arrangement on the surface of the substrate such as, for example, an array of capture agents on the surface of the substrate. The binding occurrences may be determined and related to the presence and/or amount of the different target proteins in a sample suspected of containing the target proteins..

As used herein, the term "binding" refers to an interaction or complexation between a portion of a target protein and at least a portion of the capture agent resulting in a sufficiently stable complex so as to permit separation of unbound materials such as, e.g., unbound non-target proteins and the like, from the complexes under given binding complexation or reaction conditions. Binding is mediated through hydrogen bonding or other molecular forces. Specific binding involves the specific recognition of one of two different molecules for the other compared to substantially less recognition of other molecules. The two different molecules are termed "members of a specific binding pair." On the other hand, non-specific binding involves non-covalent binding between molecules that is relatively independent of specific surface structures. Non-specific binding may result from several factors including hydrophobic interactions between molecules.

The present invention provides for advantages over known methods. The present methods and devices exhibit enhanced specificity. Two or more different capture agents capture a single target protein, which reduces non-specific binding by non-target proteins and other molecules. The invention also allows improved sensitivity. The improved specificity allows the removal of irrelevant binding and, thus, consequent noise is reduced and signal to noise ratio is increased. The present devices allow much easier construction. Compared to, for example, sandwich assays, an array of heterogeneous capture agents in accordance with the present invention can be produced as easily as a single homogeneous capture agent array. The simplicity of construction can result in reduced production costs compared to solutions and protocols that require multiple capture agents in multiple formats to be produced and packaged as in, for example, sandwich assays and so forth. The ease of operation for an array with high specificity composed of multiple independent capture agents per feature is simpler than the multiple steps required for sandwich assay procedures.

The following discussion describes in detail the various reagents and surfaces that are employed in the practice of the present invention.

### Capture Agents

The capture agent may be any entity that can bind to at least a portion of the target protein, usually binding to the protein in a specific manner. The capture agent may be a polynucleotide such as a nucleic acid, an oligonucleotide such as, e.g., an aptamer, and so forth; a peptide; a polypeptide, e.g., a protein such as a recombinant engineered protein, an antibody, an enzyme, etc.; an antibody mimic, such as a Pronectin™ biopolymer, etc.; a display protein such as, e.g., a ribosomal display protein, phage display protein, and so forth; a peptide mimetic; and so forth. The capture agent may be naturally occurring or may be produced by synthetic techniques such as recombinant techniques, hybridoma techniques, fusion techniques, and so forth.

The capture agents may all be selected from the same type of entities or they may be selected from different types. For example, some of the capture agents on a surface of a substrate may be aptamers and some may be antibodies and so forth. Furthermore, it is within the purview of the invention that the capture agents for each different protein may be selected from the same entity type or from different types. One consideration for selection of the capture agents for a particular analysis of a mixture of target proteins is that the separate independent interaction with a single target protein molecule be such that the target protein in question binds to the capture agent and that other target proteins present do not bind to the capture agent to any significant degree so that the detection of the target protein in question is compromised. In one aspect of the invention, multiple heterogeneous capture agents may be employed to optimize specificity with respect to a range of operating conditions.

Aptamer: One example of a capture agent is an aptamer. As used herein, the term "aptamer" means a specific binding oligonucleotide, which is an oligonucleotide that is capable of forming a complex with an intended target protein. The complexation is target-specific in the sense that other materials such as other proteins that may accompany the target protein do not complex to the aptamer. It is recognized that complexation and affinity are a matter of degree; however, in this context, "target-specific" means that the aptamer binds to target with a much higher degree of affinity than it binds to contaminating materials. The meaning of specificity in this context is thus similar to the meaning of specificity as applied to antibodies, for example. The aptamer may be prepared by any known method, including synthetic, recombinant, and purification methods. Further, the term "aptamer" also includes "secondary aptamers" containing a consensus sequence derived from comparing two or more known aptamers to a given target.

In general, a minimum of approximately 9 nucleotides, preferably at least 35 nucleotides, is necessary to effect specific binding of the aptamer to a target protein. The only apparent limitations on the binding specificity of the target/aptamer complexes of the invention concern sufficient sequence to be distinctive in the binding oligonucleotide and sufficient binding capacity of the target substance to obtain the necessary interaction. Oligonucleotides of sequences shorter than nine may also be feasible if the appropriate interaction can be obtained in the context of the environment in which the target protein is found or placed. Although the oligonucleotides of the aptamers generally are single-stranded or double-stranded, it is contemplated that aptamers may sometimes assume triple-stranded or quadruple-stranded structures.

In accordance with the present invention, the aptamer capture agent may be selected for each protein. An example of an approach is that described in U.S. Patent Nos. 5,270,163 and 5,475,096. In addition, oligonucleotides that bind to a target protein may also be identified by *in vitro* selection. Another approach is that described by Blackwell, T. K., *et al.,* Science (1990) 250:1104-1110; Blackwell, T. K., *et al.,* Science (1990) 250:1149-1152; Tuerk, C., and Gold, L., Science (1990) 249:505-510; Joyce, G. F., Gene (1989) 82:83-87. Tuerk and Gold describe the use of a procedure termed "systematic evolution of ligands by exponential enrichment." Kinzler, K. W., *et al.,* Nucleic Acids Res. (1989) 17:3645-3653, identified double-stranded DNA sequences that were bound by proteins that bind to DNA and regulate gene expression. Thiesen, H.-J., and Bach, C., Nucleic Acids Res. (1990) 18:3203-3208, describe what they call a target detection assay (TDA) to determine double-stranded DNA binding sites for putative DNA binding proteins.

The specific binding oligonucleotides of the aptamers should contain the sequence-conferring specificity, but may be extended with flanking regions and otherwise derivatized or modified.

The aptamers found to bind to the target proteins may be isolated, sequenced, and then re-synthesized as conventional DNA or RNA moieties, or may be "modified" oligomers, which are those conventionally, recognized in the art. These modifications include, but are not limited to incorporation of: (1) modified or analogous forms of sugars (ribose and deoxyribose); (2) alternative linking groups; or (3) analogous forms of purine and pyrimidine bases.

The aptamers may also include intermediates in their synthesis. For example, any of the hydroxyl groups ordinarily present may be replaced by phosphonate groups, phosphate groups, protected by a standard protecting group, or activated to prepare additional linkages to additional nucleotides. The 5' terminal OH is conventionally free but may be phosphorylated; OH substituents at the 3' terminus may also be phosphorylated. The hydroxyls may also be derivatized to standard protecting groups.

One or more phosphodiester linkages may be replaced by alternative linking groups. These alternative linking groups include, but are not limited to embodiments wherein P(O)O is replaced by P(O)S ("thioate"), P(S)S ("dithioate"), P(O)NR₂ ("amidate"), P(O)R, P(O)OR', CO or CH₂ ("formacetal"), wherein each R or R' is independently H or substituted or unsubstituted alkyl (1-20C.) optionally containing an ether (--O--) linkage, aryl, alkenyl, cycloalkyl, cycloalkenyl or aralkyl.

The oligonucleotides of the aptamers may contain the conventional bases adenine, guanine, cytosine, and thymine or uridine. Aptamers may be sequenced and re-synthesized as mentioned above. Included within the term aptamers are synthetic aptamers that incorporate analogous forms of purines and pyrimidines. "Analogous" forms of purines and pyrimidines are those generally known in the art, many of which are used as chemotherapeutic agents. An exemplary but not exhaustive list includes aziridinylcytosine, 4-acetylcytosine, 5-fluorouracil, 5-bromouracil, 5-carboxymethylaminomethyl-2-thiouracil, 5-carboxymethyl-aminomethyluracil, inosine, N6-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyl-uracil, 5-methoxyaminomethyl-2-thiouracil, beta-D-mannosylqueosine, 5-methoxyuracil, 2-methyl-thio-N6-isopentenyladenine, uracil-5-oxyacetic acid methylester, pseudouracil, queosine, 2-thiocytosine, 5-methyl-2-thiouracil, 2-thiouracil, 4-thiouracil, 5-methyluracil, uracil-5-oxyacetic acid, 5-pentynyl-uracil and 2,6-diaminopurine. The use of uracil as a substitute base for thymine in deoxyribonucleic acid (hereinafter referred to as "dU") is considered to be an "analogous" form of pyrimidine in this invention.

Aptamer oligonucleotides may contain analogous forms of ribose or deoxyribose sugars that are generally known in the art. An exemplary, but not exhaustive list includes 2' substituted sugars such as 2'-O-methyl-, 2'-O-allyl, 2'-fluoro- or 2'-azido-ribose, carbocyclic sugar analogs, alpha-anomeric sugars, epimeric sugars such as arabinose, xyloses or lyxoses, pyranose sugars, furanose sugars, sedoheptuloses, acyclic analogs and abasic nucleoside analogs such as methyl riboside.

One particular embodiment of aptamers that are useful in the present invention is based on RNA aptamers as disclosed in U.S. Patent Nos. 5,270,163 and 5,475,096, the relevant disclosures of which are incorporated herein by reference. The aforementioned patents disclose the SELEX method, which involves selection from a mixture of candidate oligonucleotides and stepwise iterations of binding, partitioning and amplification, using the same general selection scheme, to achieve virtually any desired criterion of binding affinity and selectivity. Starting from a mixture of nucleic acids, preferably comprising a segment of randomized sequence, the SELEX method includes steps of contacting the mixture with a target such as a target protein under conditions favorable for binding, partitioning unbound nucleic acids from those nucleic acids which have bound specifically to target molecules, dissociating the nucleic acid-target complexes, amplifying the nucleic acids dissociated from the nucleic acid-target complexes to yield a ligand-enriched mixture of nucleic acids, then reiterating the steps of binding, partitioning, dissociating and amplifying through as many cycles as desired to yield highly specific, high affinity nucleic acid ligands to the target molecule.

The SELEX method encompasses the identification of high-affinity nucleic acid ligands containing modified nucleotides conferring improved characteristics on the ligand, such as improved in vivo stability or improved delivery characteristics. Examples of such modifications include chemical substitutions at the ribose and/or phosphate and/or base positions.

Another particular embodiment of aptamers that are useful in the present invention is disclosed in U.S. Patent No. 6,001,577, the relevant disclosures of which are incorporated herein by reference. Nucleic acid ligands to target molecules are identified using the SELEX procedure wherein the candidate nucleic acids contain photoreactive groups and nucleic acid ligands identified thereby. The complexes of increased affinity nucleic acids and target molecules formed in the procedure are crosslinked by irradiation to facilitate separation from unbound nucleic acids. In other methods partitioning of high and low affinity nucleic acids is facilitated by primer extension steps in which chain termination nucleotides, digestion resistant nucleotides or nucleotides that allow retention of the cDNA product on an affinity matrix are differentially incorporated into the cDNA products of either the high or low affinity nucleic acids and the cDNA products are treated accordingly to amplification, enzymatic or chemical digestion or by contact with an affinity matrix.

Antibodies: An antibody is an immunoglobulin that specifically binds to and is thereby defined as complementary with a particular spatial and polar organization of another molecule. The antibody can be monoclonal or polyclonal and can be prepared by techniques that are well known in the art such as immunization of a host and collection of sera (polyclonal) or by preparing continuous hybrid cell lines and collecting the secreted protein (monoclonal), or by cloning and expressing nucleotide sequences or mutagenized versions thereof coding at least for the amino acid sequences required for specific binding of natural antibodies. Antibodies may include a complete immunoglobulin or fragment thereof, which immunoglobulins include the various classes and isotypes, such as IgA, IgD, IgE, IgG1, IgG2a, IgG2b and IgG3, IgM, etc. Fragments thereof may include Fab, Fv (including single chain Fv (scFv)) and F(ab')₂, Fab', and the like. In addition, aggregates, polymers, and conjugates of immunoglobulins or their fragments can be used where appropriate so long as binding affinity for a particular molecule is maintained.

Antiserum containing antibodies (polyclonal) is obtained by well-established techniques involving immunization of an animal, such as a rabbit, guinea pig, or goat, with an appropriate immunogen and obtaining antisera from the blood of the immunized animal after an appropriate waiting period. Reviews are provided by Parker, Radioimmunoassay of Biologically Active Compounds, Prentice-Hall (Englewood Cliffs, N.J., U.S., 1976), Butler, J. Immunol. Meth. 7: 1-24 (1975); Broughton and Strong, Clin. Chem. 22: 726-732 (1976); and Playfair, et al., Br. Med. Bull. 30: 24-31 (1974).

Antibodies can also be obtained by somatic cell hybridization techniques, such antibodies being commonly referred to as monoclonal antibodies. Monoclonal antibodies may be produced according to the standard techniques of Köhler and Milstein, Nature 265:495-497, 1975. Reviews of monoclonal antibody techniques are found in Lymphocyte Hybridomas, ed. Melchers, et al. Springer-Verlag (New York 1978), Nature 266: 495 (1977), Science 208: 692 (1980), and Methods of Enzymology 73 (Part B): 3-46 (1981). Samples of an appropriate immunogen preparation are injected into an animal such as a mouse and, after a sufficient time, the animal is sacrificed and spleen cells obtained. Alternatively, the spleen cells of a non-immunized animal can be sensitized to the immunogen in vitro. The spleen cells comprising the chromosomes encoding the base sequences for the desired immunoglobins can be fused with a myeloma cell line, generally in the presence of a non-ionic detergent, for example, polyethylene glycol. The resulting cells, which include fused hybridomas, are allowed to grow in a selective medium, such as HAT-medium, and the surviving immortalized cells are grown in such medium using limiting dilution conditions. The cells are grown in a suitable container, e.g., microtiter wells, and the supernatant is screened for monoclonal antibodies having the desired specificity.

Various techniques exist for enhancing yields of monoclonal antibodies, such as injection of the hybridoma cells into the peritoneal cavity of a mammalian host, which accepts the cells, and harvesting the ascites fluid. Where an insufficient amount of the monoclonal antibody collects in the ascites fluid, the antibody is harvested from the blood of the host. Alternatively, the cell producing the desired antibody can be grown in a hollow fiber cell culture device or a spinner flask device, both of which are well known in the art. Various conventional ways exist for isolation and purification of the monoclonal antibodies from other proteins and other contaminants (see Köhler and Milstein, supra).

In another approach for the preparation of antibodies the sequence coding for antibody binding sites can be excised from the chromosome DNA and inserted into a cloning vector, which can be expressed in bacteria to produce recombinant proteins having the corresponding antibody binding sites.

In general, antibodies can be purified by known techniques such as chromatography, e.g., DEAE chromatography, ABx chromatography, and the like, filtration, and so forth.

Display Proteins: Another family of proteins for the capture agents of the invention is display proteins preferably without their attendant nucleic acid portion. One example of such a display protein is ribosomal display proteins, which generally comprise one or more proteins initially associated with an RNA molecule. Ribosomes are ribonucleoprotein particles in which rRNA provides a majority of the mass. In the present invention capture agents may be selected from a library of expressed proteins that are no longer attached to their encoding message through the ribosomal complex.

Another example of display proteins is phage display proteins in which the encoding message is integrated into a filamentous bacteriophage genome and the resulting expressed protein is displayed on the surface of the head of the virus. The displayed protein can be any desired sequence, but is most commonly an antibody, antibody fragment or a combinatorial peptide.

Synthetic affinity reagents: Another family of capture agents is synthetic affinity reagents, which include any reagent that is synthesized (as opposed to isolated from natural sources) and that has an affinity for the target proteins. Examples of such affinity reagents, by way of illustration and not limitation, are biomimetics, imprinted polymeric substrates, small molecule ligand partners, and the like.

Biomimetics are any synthetic organic molecule that has been constructed to "mimic" a biologically inspired binding epitope. Included within this group are antibody mimics, peptide mimetics, small organic moieties based on a DNA motif, for example, to capture DNA binding proteins, and the like.

Antibody Mimics: Chemicals that mimic the functions of antibodies are known. Such antibody mimics usually are small in size, which allows them to avoid provoking an immunogenic response. There are several approaches to the structure and manufacture of antibody mimics. One approach utilizes an alternative protein framework, such as cytochrome b₅₆₂. (Hsieh-Wilson, *et al.,* (1996), Acc. Chem Res. 29:164-170). Some of the functions of antibodies have been mimicked in structures comprising ribonucleic acids (RNA). Hsieh-Wilson, et al., (1996), supra).

Unnatural oligomers such as benzodiazepines, beta-turn mimics, protease inhibitors and purine derivatives have also been tested for their ability to function as antibody mimics. (Hsieh-Wilson, et al., (1996), supra). Unnatural biopolymers such as oligocarbamates, oligoureas and oligosulfones have been proposed as antibody mimics. (Hsieh-Wilson, et al., (1996), supra).

Molecules with some of the recognition properties of antibodies have been created by joining various substituents to scaffolds such as xanthene and cubane. These substituents include peptides but do not include peptide loops. (Hsieh-Wilson, et al., (1996), supra). Antibody mimics comprising a scaffold of a small molecule such as 3-aminomethylbenzoic acid and a substituent consisting of a single peptide loop have been constructed. The peptide loop performs the binding function in this mimic. (Smythe, et al., (1994), J. Am. Chem. Soc. 116:2725-2733)

Another example of an antibody mimic is that disclosed in U.S. Patent No. 5,770,380. The antibody mimic has multiple peptide loops bound to an organic or molecular scaffold. In a preferred embodiment, the loops all project from the same side of the scaffold with respect to one another. The binding sites of the antibody mimic are provided by peptide loops. The potential diversity of binding sites is alleged to be far greater than with other oligomers and polymers. Because the loops project from the same side of the scaffold, all of the loops are available for binding purposes and binding ability is increased.

Another capture agent is a Pronectin™ biopolymer, which is a synthetic antibody mimic based on fibronectins, developed by Phylos, Inc., Lexington, Massachusetts. In the process of selecting Pronectin™ biopolymers as specific capture agents, a large library of RNA is expressed and the messages are covalently attached at the 3' end to the product protein by puromycin. Pronectin-F™ biopolymer is a recombinant protein containing multiple copies of the RGD cell attachment ligand of human fibronectin interspersed between repeated structural peptide segments derived from spider silk. Pronectin F™ is a trademark of Protein Polymer Technologies of San Diego, Calif.). See, for example, U.S. Patent No. 5,514,581, the relevant disclosure of which is incorporated herein by reference.

Peptide Mimetics: Another family of capture agents includes peptide mimetics, i.e., nonpeptide synthetic polymers or oligomers that detectably interact with proteins or receptors in a manner analogous to protein-protein or protein-peptide physical and/or chemical interactions under assay conditions. Peptide mimetics are generally protease-resistant, and include, for example, oligomeric species such as peptoids, beta-peptides, and constrained cyclic molecules such as cyclic peptides and heterocyles. In some cases, the peptide mimetic may also mimic the folding of natural proteins. Peptide mimetics may comprise generally no more than about 500 mers, no more than about 100 mers, no more than about 50 mers, no more than about 40 mers, no more than about 30 mers, no more than about 20 mers, no more than about 10 mers, no more than about 7 mers, and may be about 7 to about 500 mers. See, for example, U.S. Patent Publication No. 20020055125 and references disclosed therein relating to peptide mimetic libraries and their design and synthesis, the relevant disclosures of which is incorporated herein by reference.

Imprinted polymeric substrates: Molecular imprinting methods are known. See, for instance, Molecular Imprinting, Macromol. Chem., 187:687 (1981). Molecular imprinting creates specific recognition sites in materials, such as polymeric organic materials. Known molecular imprinting techniques involve crosslinking materials in the presence of a functional monomer or mixture of monomers. The imprinting molecule interacts with a complementary portion of a functional monomer, either covalently or by other interactions such as ionic, hydrophobic or hydrogen bonding, so that recognition sites for the imprinting molecule can be provided in the substrate material. The imprinting molecule is then removed from the substrate to leave the recognition site. See, for example, U.S. Patent No. 5,110,883 (the relevant disclosures of which are incorporated herein by reference) describes the preparation of synthetic enzymes and synthetic antibodies by molecular imprinting techniques.

### Solid Substrate

The capture agents are bound to the surface of a solid substrate. In one embodiment, the capture agents are bound to the surface of the substrate in a predetermined arrangement or pattern, which means any arrangement on the surface where the identity of a capture agent at a particular location is known. In one embodiment the predetermined arrangement is an array as discussed more fully herein.

Preferred materials for the substrate itself are those that provide physical support for the capture agents that are bound on the surface. The materials should be of such a composition that they endure the conditions of binding of the capture agents to the surface of the substrate and of any subsequent treatment or handling or processing that may be encountered in the use of the particular substrate.

In one embodiment, the substrate material is transparent. By "transparent" is meant that the substrate material permits signal from features on the surface of the substrate to pass therethrough without substantial attenuation and also permits any interrogating radiation to pass therethrough without substantial attenuation. By "without substantial attenuation" may include, for example, without a loss of more than 40% or more preferably without a loss of more than 30%, 20% or 10%, of signal. The interrogating radiation and signal may be, for example, visible, ultraviolet or infrared light. The materials from which the substrate may be fabricated should ideally exhibit a low level of non-specific binding during hybridization events.

The materials may be naturally occurring or synthetic or modified naturally occurring. Suitable rigid substrates may include glass, which term is used to include silica, and include, for example, glass such as glass available as Bioglass, and suitable plastics. Additional rigid, non-transparent materials may be considered, such as silicon, mirrored surfaces, laminates, ceramics, opaque plastics, such as, for example, polymers such as, e.g., poly (vinyl chloride), polyacrylamide, polyacrylate, polyethylene, polypropylene, poly(4-methylbutene), polystyrene, polymethacrylate, poly(ethylene terephthalate), nylon, poly(vinyl butyrate), etc., either used by themselves or in conjunction with other materials. The surface of the substrate is usually the outer portion of a substrate.

The material used for a substrate may take any of a variety of configurations ranging from simple to complex. The support can have any one of a number of shapes, such as strip, plate, disk, rod, particle, including bead, tube, well, and the like. Usually, the material is relatively planar such as, for example, a slide. In many embodiments, the material is shaped generally as a rectangular solid. Multiple predetermined arrangements such as, e.g., arrays of capture agents, may be synthesized on a sheet, which is then diced, i.e., cut by breaking along score lines, into single array substrates. Typically, in one embodiment the substrate has a length in the range about 5 mm to 100 cm, usually about 10 mm to 25 cm, more usually about 10 mm to 15 cm, and a width in the range about 4 mm to 25 cm, usually about 4 mm to 10 cm and more usually about 5 mm to 5 cm. The substrate may have a thickness of less than 1 cm, or even less than 5 mm, 2 mm, 1 mm, or in some embodiments even less than 0.5 mm or 0.2 mm. The thickness of the substrate is about 0.01 mm to 5.0 mm, usually from about 0.1 mm to 2 mm and more usually from about 0.2 to 1 mm. The substrate is usually cut into individual test pieces, which may be the size of a standard size microscope slide, usually about 3 inches in length and 1 inch in width.

The surface of the substrate onto which the capture agents are bound may be smooth or substantially planar, or have irregularities, such as depressions or elevations. The surface may be modified with one or more different layers of compounds that serve to modify the properties of the surface in a desirable manner. Such modification layers, when present, will generally range in thickness from a monomolecular thickness to about 1 mm, usually from a monomolecular thickness to about 0.1 mm and more usually from a monomolecular thickness to about 0.001 mm. Modification layers of interest include inorganic and organic layers such as metals, metal oxides, polymers, small organic molecules and the like. Polymeric layers of interest include polysaccharides, phospholipids, polyurethanes, polyesters, polycarbonates, polyureas, polyamides, polyethylene amines, polyarylene sulfides, polysiloxanes, polyimides, polyacetates, and the like, where the polymers may be hetero- or homo-polymeric, and may or may not have separate functional moieties attached thereto (for example, conjugated).

The capture agents may be bound to the surface of the substrate in any manner as long as the capture agents are bound non-diffusively to the surface. That is, the capture agents must not diffuse from the surface particularly under the conditions of the methods of the invention. In other words, the capture agents should be bound to the surface of the substrate in such a manner that they do not become unbound during subsequent incubation and processing steps in accordance with the invention such as washing the surface to remove unbound target proteins. The capture agents may be bound by being non-covalently linked, e.g., adhered, absorbed, adsorbed, or by being covalently-linked to the surface of the substrate. For covalent linking the surface of the substrate contains functional groups for linking of the capture agents. The nature of the functional groups is dependent on the nature of the capture agents.

A variety of methods have been reported for the covalent attachment of molecules to a surface. Typically, these reactions are performed by the reaction of an active functional group on a molecule with an activated functional group on the surface. As an example, an amine-containing compound can be attached to a carboxylic acid containing surface by forming an activated ester of the carboxylic acid, such as an N-hydroxysuccinimide derivative. The amine readily reacts with this activated ester to form a stable amide bond. This reaction is useful under conditions whereby the reaction with the desired amine is significantly faster than with other nucleophiles in the system.

Examples of methods that have been previously described include the activation of surfaces with cyanogen bromide, N-hydroxysuccinimide esters, carbonyl diimidazole, carbodiimides, azlactones, cyanuric chlorides, organic sulfonyl chlorides, divinyl sulphone, nitrophenyl esters, iodoacetyl, maleimide, epoxy, hydrazide, reductive amination, diazonium salts and Mannich condensations. Molecules that react with the activated surface include amines, alcohols, carboxylic acids, thiols, carbonyls, and compounds containing active hydrogens.

The capture agent can be attached directly to the surface of the substrate or it can be attached by means of a linking group. The linking group may be a chain of from 1 to 100 atoms, usually from about 1 to 70 atoms, preferably, 1 to 50 atoms, more preferably 1 to 20 atoms, each independently selected from the group normally consisting of carbon, oxygen, sulfur, nitrogen, halogen and phosphorous. The number of heteroatoms in the linking groups will normally range from about 0 to 20, usually from about 1 to 15, more preferably 2 to 6. The atoms in the chain may be substituted with atoms other than hydrogen, which may be a single atom such as a halogen, etc., or part of a group of atoms forming a functionality such as oxo or oxy, nitro, nitroso or amino, normally bonded to carbon, phosphonate and phosphate mono- or diester. The linking groups may be aliphatic or aromatic.

For the most part, a linking group prior to linking will have a functionality for linking such as a non-oxocarbonyl group including nitrogen and sulfur analogs, a phosphate group, an amino group, alkylating agents such as halo or tosylalkyl, oxy (hydroxyl or the sulfur analog, mercapto) oxocarbonyl (e.g., aldehyde or ketone), or active olefin such as a vinyl sulfone or α-, β-unsaturated ester. These functionalities will be linked to amine groups, carboxyl groups, nucleophiles (e.g. hydroxyl, thiol). Where an amine and carboxylic acid or its nitrogen derivative or phosphoric acid are linked, amides, amidines and phosphoramides will be formed. Where mercaptan and activated olefin are linked, thioethers will be formed. Where a mercaptan and an alkylating agent are linked, thioethers will be formed. Where aldehyde and an amine are linked under reducing conditions, an alkylamine will be formed. Where a carboxylic acid or phosphate acid and an alcohol are linked, esters will be formed.

The composition of the linking group is not critical to the present invention. It is often preferred that the linking group be hydrophilic or aqueous friendly. As a general rule, the length of a particular linking group can be selected arbitrarily to provide for convenience of synthesis and the incorporation of the desired capture agents at the positions and distances required for the binding of two or more capture agents to a molecule of target protein. The linking group should not contain functionalities, or be of a length, that will interfere with the reactions that are desired in accordance with the present invention. Furthermore, the chemistry used to introduce the linking group should not be detrimental to the molecule in question. The linking group may be bi-functional, i.e., having a functionality for linking to the surface and a functionality for linking to the capture agent.

As mentioned above, the present invention employs a set of capture agents for each target protein. Each set of capture agents may include at least two, at least three, at least four, etc., capture agents depending on the nature of the capture agents and the nature and number of the target proteins, the level of sensitivity and specificity desired for any particular determination, cost considerations, total number of targets, and the like.

The capture agents for each set of capture agents are spatially disposed on the surface of the substrate in manner such that the capture agents of the set bind to a single molecule of the target protein. Accordingly, the capture agents of a particular set exhibit multidentate (for example, bidentate, tridentate and the like) binding to the molecule of target protein that corresponds to the particular set of capture agents. The spatial disposition, on the surface of the substrate, of the capture agents of a set is dependent on the nature of the target protein, the binding affinity of each of the capture agents for the respective portion of the target protein to which the capture agents bind, and so forth. The spatial disposition of the capture agents on the surface of the substrate may be determined empirically.

In one approach the capture agents of a set may be linked together to form a conjugate capture agent comprising the members of the set that bind in a multidentate manner to a molecule of the target protein. On the other hand, the conjugate capture agent may naturally comprise multiple portions that bind to different portions of the molecule of target protein. The conjugate capture agent may be bound to the surface of the substrate in manner as described above. The members of the conjugate capture agent are linked together so that their binding to the respective portions of the molecule of the target protein is subject to many of the considerations as mentioned above for the spatial disposition of the members of the set on the surface of the substrate.

Where the conjugate capture agent is formed by linking two or more capture agent moieties together, the linking may be by any of the approaches discussed above for linking the capture agents to the surface of the substrate. Typically, two or more capture agents may be linked together by the use of a scaffold that includes a spacer moiety that provides the relative constraint necessary to present the capture agents to the target protein binding sites. In other words, the spacer provides for appropriate distances for reaction of multiple different capture agents of a set with a single molecule of target protein. The spacer moiety will usually comprise two or more functionalities for linking to the respective capture agents. The functionalities include those discussed hereinabove. The length of the spacer is dependent on the nature of the target protein including the relevant binding sites, the nature of the capture agents including the affinity of binding between the capture agents and the target protein binding sites, the number of capture agents for each target protein, the composition of the linking group, and so forth. The length of the spacer is generally that which permits the binding of the linked capture agents to the same molecule of target protein. In many circumstances the length of the spacer is determined empirically.

Polynucleotides that serve as capture agents for the target proteins may be deposited on the surface of a solid substrate as fully formed moieties. On the other hand, the polynucleotides may be synthesized *in situ* in a series of steps such as, for example, the addition of nucleotide building blocks. Such methods of fabrication are well known. See, for example, EP 0 173 356 B1, U.S. Patent No. 5,700,637 and PCT WO 95/25116 and PCT application WO 89/10977, U.S. Patent No. 6,242,266 (Schleifer, *et al.),* (U.S. Patent No. 6,232,072) (Fisher), U.S. Patent No. 6,180,351 (Cattell), U.S. Patent No. 5,474,796 (Brennan), U.S. Patent No. 6,219,674 (Fulcrand, *et al.),* U.S. Patent No. 6,258,454 (Lefkowitz, *et al.).*

The devices and methods of the present invention are particularly useful for arrays of capture agents on the surface of the substrate. An array includes any one-, two- or three- dimensional arrangement of addressable regions bearing a particular capture agent associated with that region. An array is addressable in that it has multiple regions of different capture agents, such that a region or feature or spot of the array at a particular predetermined location or address on the array can detect a particular target protein by virtue of binding specifically to such target protein.

An array assembly on the surface of a substrate refers to one or more arrays disposed along a surface of an individual substrate and separated by inter-array areas. Normally, the surface of the substrate opposite the surface with the arrays (opposing surface) does not carry any arrays. The arrays can be designed for testing against any type of sample, whether a trial sample, a reference sample, a combination of the foregoing, or a known mixture of proteins. The surface of the substrate may carry at least one, two, four, or at least ten, arrays. Depending upon intended use, any or all of the arrays may be the same or different from one another and each may contain multiple spots or features of capture agents. A typical array may contain more than ten, more than one hundred, more than one thousand or ten thousand features, or even more than one hundred thousand features, in an area of less than 20 cm² or even less than 10 cm². For example, features may have widths (that is, diameter, for a round spot) in the range from a 10 µm to 1.0 cm. In other embodiments each feature may have a width in the range of 1.0 µm to 1.0 mm, usually 5.0 µm to 500 µm, and more usually 10 µm to 200 µm. Non-round features may have area ranges equivalent to that of circular features with the foregoing width (diameter) ranges.

Any of a variety of geometries of arrays on a substrate may be used. As mentioned above, an individual substrate may contain a single array or multiple arrays. Features of the array may be arranged in rectilinear rows and columns. This is particularly attractive for single arrays on a substrate. When multiple arrays are present, such arrays can be arranged, for example, in a sequence of curvilinear rows across the substrate surface (for instance, a sequence of concentric circles or semi-circles of spots), and the like. Similarly, the pattern of features may be varied from the rectilinear rows and columns of spots to include, for example, a sequence of curvilinear rows across the substrate surface (for example, a sequence of concentric circles or semi-circles of spots), and the like. The configuration of the arrays and their features may be selected according to manufacturing, handling, and use considerations.

Each feature, or element, within the molecular array is defined to be a small, regularly shaped region of the surface of the substrate. The features are arranged in a predetermined manner. Each feature of an array usually carries a predetermined capture agent or mixtures thereof. Each feature within the molecular array may contain a different molecular species, and the molecular species within a given feature may differ from the molecular species within the remaining features of the molecular array. Some or all of the features may be of different compositions. Each array may contain multiple spots or features and each array may be separated by spaces or areas. It will also be appreciated that there need not be any space separating arrays from one another. Interarray areas and interfeature areas are usually present but are not essential. As with any border areas, these interarray and interfeature areas do not carry any capture agents. It will be appreciated that the interarray areas and interfeature areas, when present, could be of various sizes and configurations.

### Methods Using the Capture Agents of the Invention

The following general discussion of methods is by way of illustration and not limitation. One skilled in the art will be able to apply the technology herein in other methods for conducting protein assays. In general, in the present methods a sample to be analyzed is contacted with the surface of a substrate comprising the plurality of sets of capture agents. The sample may be a body fluid or a non-body fluid. The phrase "body fluid" refers to any fluid obtained from the body of a mammal (e.g., human, monkey, mouse, rat, rabbit, dog, cat, sheep, cow, pig, and the like) that is suspected of containing a particular target protein or proteins to be detected. Body fluids include, for example, whole-blood, plasma, serum, interstitial fluid, sweat, saliva, urine, semen, blister fluid, inflammatory exudates, stool, sputum, cerebral spinal fluid, tears, mucus, and the like, collection fluids used to collect proteins from protein-containing materials such as biological tissue and the like, and so forth. The biological tissue includes excised tissue from an organ or other body part of a host.

The phrase "non-body fluid" refers to any fluid not obtained from the body of a mammal, which is suspected of containing a particular target protein or proteins to be detected. Exemplary non-body fluids include cell-culture media, dialysate, and the like. The protein sample can be examined directly or may be pretreated to render the protein analyte more readily accessible to the protein-binding moiety of the capture agents of the invention.

For determining a mixture of proteins, one may use intact cells, intact viruses, viral infected cells, lysates, plasmids, mitochondria or other organelles, fractionated samples, or other aggregation of proteins, separated proteins, and treated proteins, by themselves or in conjunction with other compounds. Any source of a mixture of proteins can be used, where there is an interest in identifying a plurality of proteins. Protein analytes may be isolated using precipitation, extraction, and chromatography. The proteins may be present as individual proteins or combined in various aggregations, such as organelles, cells, viruses, etc. Protein analytes may be released from cells, for example, by lysing the cells.

As mentioned above, in the method, the sample in a suitable assay medium is contacted with multiple sets of capture agents bound to the surface of a substrate where each set of capture agents is selected to bind a particular protein of interest. The medium is usually an aqueous buffered medium at a moderate pH, generally that which provides optimum sensitivity in the present method. The aqueous medium may be solely water or may include from 0.01 to 80 or more volume percent of a cosolvent. The pH for the medium will usually be in the range of about 4 to 13, more usually in the range of about 5 to 10, and preferably in the range of about 6.5 to 9.5. The pH is generally selected to achieve optimum assay sensitivity and specificity. Among the factors that must be considered are the pH dependence of the rates of the reactions involved, the binding of binding members and the minimization of non-specific binding, and so forth.

Various buffers may be used to achieve the desired pH and maintain the pH during the determination. Illustrative buffers include borate, phosphate, carbonate, tris, barbital and the like. The particular buffer employed is not critical to this invention, but in an individual assay one or another buffer may be preferred. Various ancillary materials may be employed in the method in accordance with the present invention. For example, in addition to buffers the medium may comprise stabilizers for the medium and for the reagents employed. Frequently, in addition to these additives, proteins may be included, such as albumins; organic solvents such as formamide; quaternary ammonium salts; polyanions such as dextran sulfate; surfactants, particularly non-ionic surfactants; binding enhancers, e.g., polyalkylene glycols; or the like.

One or more incubation periods may be employed. The medium is usually incubated with the surface of the substrate at a temperature and for a time sufficient for binding of the sets of capture agents to the respective target protein that may be present in the sample. The incubation period is generally carried out under conditions sufficient to ensure an efficient and quantitatively reproducible binding equilibrium between the aforementioned species. Moderate temperatures are normally employed for carrying out the method and usually constant temperature, preferably, room temperature, during the period of the measurement. Incubation temperatures normally range from about 5° to about 99°C, usually from about 15°C to about 70°C, more usually 20°C to about 45°C. The time period for the incubation is dependent on, for example, the kinetics of the reactions between the capture agents and the target proteins, and so forth and is about 0.2 seconds to about 12 hours, usually, from about 2 seconds to 1 hour, more usually, about 10 to about 30 minutes. The time period depends on, among others, the temperature of the medium and the rate of binding of the target proteins and capture agents, which is determined by the association rate constant, the concentration, the binding constant and dissociation rate constant.

The concentration of proteins to be detected will generally vary from about 10⁻¹⁵ M to about 10⁻¹² M, more usually from about 10⁻¹⁵ M to about 10⁻¹⁴ M. In some circumstances, a predetermined cut-off level may be established for one or more of the protein analytes suspected of being in a sample. The particular predetermined cut-off level generally is determined on a case-by-case basis by one skilled in the art.

The concentration of the capture agents is dependent on the suspected concentrations of the proteins of interest in the sample, the stability of the capture agent, the characteristics of the binding surfaces, characteristics of the detection system, and so forth. The final concentration of any of the reagents will normally be determined empirically to optimize the sensitivity of the method. The concentration of the capture agents is generally about picomolar to nanomolar.

Following the incubation period(s), the surface of the substrate is examined for the presence and/or amount of target proteins. As mentioned above, the usually each member of the set of capture agents for a particular target protein bind to the same molecule of target protein. Again, as explained above, the spatial disposition of the capture agents on the surface of the substrate can assist in determining the binding of each member of a set of capture agents to the same molecule of target protein.

Usually, the surface of the substrate is washed with a suitable wash solution such as a buffered solution to remove unbound materials. Any convenient wash solution may be employed to separate the bound and unbound materials. The conditions for washing the surface should not result in denaturing the complexes of capture agents and target proteins. The wash medium may be an aqueous medium as discussed above. For non-denaturing conditions the pH of the medium is usually about 6.5 to about 7.5, the temperature of the wash medium is usually about 25 to about 60°C, usually about 37°C, and time period is usually about 10 minutes to about 1 hour. To avoid denaturing, the aqueous wash medium should not contain urea, guanidine, detergents, or organic solvents, and so forth in an amount sufficient to denature the complexes. The resulting surface contains only those target proteins from the original sample that correspond to particular sets of capture agents.

In one approach to determining that each member of a set of capture agents binds to the same molecule of target protein, the stringency of the wash conditions may be adjusted. In this approach wash conditions are chosen so that a molecule of target protein that binds to only one member of the set of capture agents is removed during the wash and a molecule of target protein that binds to all members of the set of capture agents is retained on the surface. Such stringency conditions include salt concentration, temperature, co-solvents such as organic solvents, competitive displacers and so forth. In general, more stringent conditions favor retention of a single molecule of target protein bound to all members of the set of capture agents and removal of a single molecule of target protein bound to less than all the members of the set of capture agents. Increased stringency is usually achieved by elevating the temperature, increasing the ratio of co-solvents, lowering the salt concentration, and the like. In addition to the above, the stringency conditions are dependent on, for example, the strength of the binding of the target proteins to the capture agents, strength of binding of the target to multiple binding agents simultaneously, the strength of non-specific binding of non-target proteins to the array substrate surface, capture agents or bound target proteins, and so forth. Usually the stringency conditions are chosen empirically.

In one embodiment for detection, the surface is contacted with a mixture of specific binding partners, each specific for a target protein suspected of being present. The specific binding partners may be selected from the same group of moieties as the capture agents. Of particular interest are antibodies, antibody fragments, synthetic antibody mimetics, biomimetics, aptamers, molecular imprinted ligands, and so forth. The specific binding partners are conjugated or linked to a member of a signal producing system, usually, a label. The linking of the specific binding partners to the label may be carried out by any of the aforementioned methods for linking the capture agents to the surface of the substrate. The labels employed may be those that are known in the art for multiplexed detection of multiple analytes.

The label is capable of being detected directly or indirectly. In general, any reporter molecule that is detectable can be a label. Labels include, for example, (i) reporter molecules that can be detected directly by virtue of generating a signal, (ii) specific binding pair members that may be detected indirectly by subsequent binding to a cognate that contains a reporter molecule, (iii) mass tags detectable by mass spectrometry, (iv) oligonucleotide primers that can provide a template for amplification or ligation and (v) a specific polynucleotide sequence or recognition sequence that can act as a ligand such as for a repressor protein, wherein in the latter two instances the oligonucleotide primer or repressor protein will have, or be capable of having, a reporter molecule and so forth. The reporter molecule can be a catalyst, such as an enzyme, a polynucleotide coding for a catalyst, promoter, dye, fluorescent molecule, chemiluminescent molecule, coenzyme, enzyme substrate, radioactive group, a small organic molecule, amplifiable polynucleotide sequence, a particle such as latex or carbon particle, metal sol, crystallite, liposome, cell, etc., which may or may not be further labeled with a dye, catalyst or other detectable group, a mass tag that alters the weight of the molecule to which it is conjugated for mass spectrometry purposes, and the like. Preferably, the label is selected from electromagnetic or electrochemical materials. In a particularly preferred embodiment, the detectable label is a fluorescent dye. Other labels and labeling schemes will be evident to one skilled in the art base on the disclosure herein.

Considerations such as the nature of the signal producing system and the nature of, and predetermined cut-off levels for, the target proteins, biological significance of specific target levels, and so forth normally determine the concentrations of the various reagents. The final concentration of each of the reagents will normally be determined empirically to optimize the sensitivity of the method. The concentration of the binding partners for each of the target proteins is usually sufficient to bind at least 1%, preferably at least 10%, more preferably, at least 90%, of the target protein.

After the appropriate period of time of contact between the solid surface and the specific binding partners, the contact is discontinued. The solid surface may be washed to remove unbound materials.

Activation of the signal producing system depends on the nature of the signal producing system members. For example, for those members of a signal producing system that are activated with light, the member is irradiated with light. Other activation methods will be suggested to those skilled in the art in view of the disclosures herein. For some signal producing systems no agent for activation is necessary such as those systems that involve a label that is a radioactive label, an enzyme, and so forth. For enzyme systems addition of a substrate and/or a cofactor may be necessary.

The examination for presence and amount of the signal also includes the detection of the signal, which is generally merely a step in which the signal is read. The signal is normally read using an instrument, the nature of which depends on the nature of the signal. The instrument may be a spectrophotometer, fluorometer, an absorption spectrometer, luminometer, chemiluminometer, actinometer, photographic instrument, and the like. The presence and amount of signal detected is related to the presence and amount of any analyte present in a sample above the predetermined cut-off level. Temperatures during measurements generally range from about 10° to about 70°C, more usually from about 20° to about 45°C, more usually about 20° to about 25°C. In one approach standard curves are formed using known concentrations of the analytes to be screened. Calibrators and other controls may also be used.

In one specific embodiment, the substrate is moved to an examining device where the surface of the substrate on which the plurality of target proteins are present, preferably in the form of an array, is interrogated. The examining device may be a scanning device involving an optical system.

Reading of the array may be accomplished by illuminating the array and reading the location and intensity of resulting fluorescence at each feature of the array. For example, a scanner may be used for this purpose where the scanner may be similar to, for example, the AGILENT MICROARRAY SCANNER available from Agilent Technologies Inc, Palo Alto, CA. Other suitable apparatus and methods are described in U.S. patent applications: Serial No. 09/846,125 "Reading Multi-Featured Arrays" by Dorsel, *et al.;* U.S. Patent No. 6,406,849, the relevant portions of which are incorporated herein by reference. However, arrays may be read by methods or apparatus other than the foregoing, with other reading methods including other optical techniques (for example, detecting chemiluminescent or electroluminescent labels) or electrical techniques (where each feature is provided with an electrode to detect complex formation at that feature in a manner disclosed in U.S. Patent Nos. 6,251,685 and 6,221,583 and elsewhere).

Results from the reading may be raw results (such as fluorescence intensity readings for each feature in one or more color channels) or may be processed results such as obtained by rejecting a reading for a feature that is below a predetermined threshold and/or forming conclusions based on the pattern rcad from the array (such as whether or not a particular target protein may have been present in the sample). The results of the reading (processed or not) may be forwarded (such as by communication) to a remote location if desired, and received there for further use (such as further processing).

In another particular embodiment, the method is carried out under computer control, that is, with the aid of a computer. For example, an IBM® compatible personal computer (PC) may be utilized. The computer is driven by software specific to the methods described herein. The preferred computer hardware capable of assisting in the operation of the methods in accordance with the present invention involves a system with at least the following specifications: Pentium® processor or better with a clock speed of at least 100 MHz, at least 32 megabytes of random access memory (RAM) and at least 80 megabytes of virtual memory, running under either the Windows 95 or Windows NT 4.0 operating system (or successor thereof).

Software that may be used to carry out the methods may be, for example, Microsoft Excel or Microsoft Access, suitably extended via user-written functions and templates, and linked when necessary to stand-alone programs that may be desired. Examples of software or computer programs used in assisting in conducting the present methods may be written, preferably, in Visual BASIC, FORTRAN and C⁺⁺, as exemplified below in the Examples. It should be understood that the above computer information and the software used herein are by way of example and not limitation. The present methods may be adapted to other computers and software. Other languages that may be used include, for example, PASCAL, PERL or assembly language.

A computer program may be utilized to carry out one or more steps of the present invention. Another aspect of the present invention is a computer program product comprising a computer readable storage medium having a computer program stored thereon which, when loaded into a computer, performs the aforementioned method.

One aspect of the invention is the product of the above method, namely, the assay result, which may be evaluated at the site of the testing or it may be shipped to another site for evaluation and communication to an interested party at a remote location if desired. By the term "remote location" is meant a location that is physically different than that at which the results are obtained. Accordingly, the results may be sent to a different room, a different building, a different part of city, a different city, and so forth. Usually, the remote location is at least about one mile, usually, at least ten miles, more usually about a hundred miles, or more from the location at which the results are obtained. The data may be transmitted by standard means such as, e.g., facsimile, mail, overnight delivery, e-mail, voice mail, and the like.

"Communicating" information references transmitting the data representing that information as electrical signals over a suitable communication channel (for example, a private or public network). "Forwarding" an item refers to any means of getting that item from one location to the next, whether by physically transporting that item or otherwise (where that is possible) and includes, at least in the case of data, physically transporting a medium carrying the data or communicating the data.

In an alternative approach to detection of the target proteins on the surface of the substrate, the target proteins may be released from the surface of the substrate following the washing step after the initial incubation with the capture agents. The target proteins may then be detected using, for example, labeled specific binding partners as discussed above. In one instance the capture agents may be linked to the surface of the substrate by a cleavable linker. After the incubation between the surface and the target proteins and subsequent washing, the surface may be treated to cleave the linker and release the target proteins for detection. Such cleavable linkers are known in the art.
In yet another alternative approach, the target proteins on the surface of the substrate may be released by increasing wash stringency, such as salt changes, pH changes etc.

Another embodiment of the present invention is a device for determining one or more different target proteins in a sample suspected of containing a plurality of the target proteins. The apparatus comprises a solid substrate comprising a surface and an array of capture agents bound to the surface. At least two capture agents specific for different binding sites on each of the target proteins are present on the surface. The two capture agents are spatially disposed to both bind to a single molecule of the target protein.

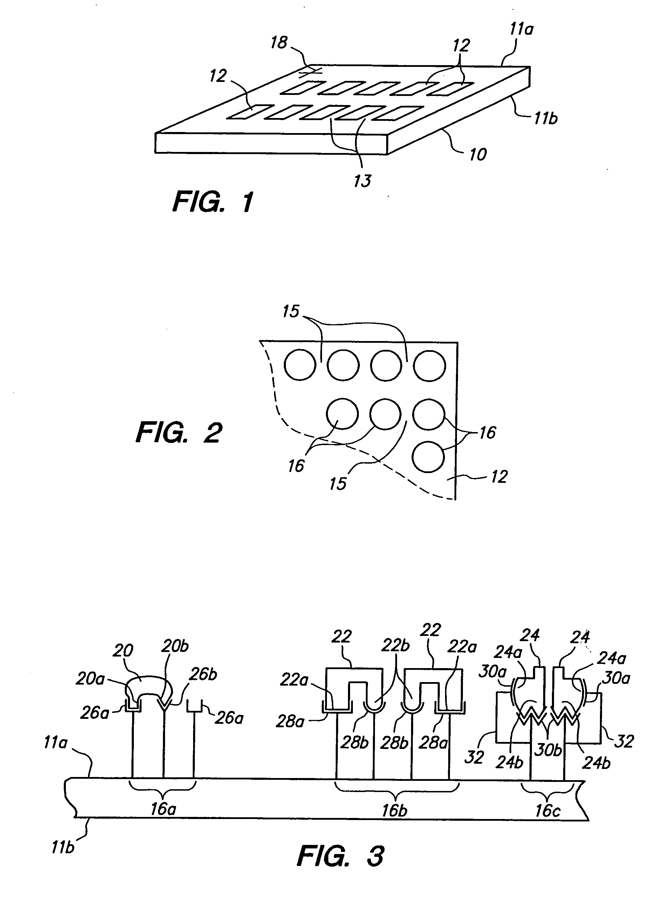

Referring to Figs. 1-3, there is shown multiple identical arrays 12 (only some of which are shown in Fig. 1), separated by inter-array regions 3, across the complete front surface 11a of a single transparent substrate 10. However, the arrays 12 on a given substrate need not be identical and some or all could be different. Each array 12 contains multiple spots or features 16 separated by inter-feature regions 15. A typical array 12 may contain from 100 to 100,000 features. Each of the features 16 represents a different set of two capture agents, one set for each of the target proteins that may be present in a sample to be analyzed. This is illustrated schematically in Fig. 3 where different regions 16 are shown as carrying different sets of two capture agents. Region 16a has a set of capture agents 26a and 26b for epitopes 20a and 20b, respectively, of target protein 20; region 16b has a set of capture agents 28a and 28b for epitopes 22a and 22b of target protein 22; and region 16c has a set of capture agents 30a and 30b for epitopes 24a and 24b, respectively, of target protein 24. As can be seen, capture agents 26a and 26b bind to respective epitopes 20a and 20b of target protein 20. In a similar manner, capture agents 28a and 28b bind to respective epitopes 22a and 22b of target protein 22. As can be seen, capture agents 30a and 30b are linked together by spacer arm 32, which is bound to surface 11a. Capture agents 30a and 30b bind to respective epitopes 24a and 24b of target protein 24. Subsequent to an incubation period for binding of capture agents to target proteins, surface 11 a is washed to remove unbound materials. Then, labeled binding partners for each of the target proteins are contacted with surface 11 a, which is subsequently washed and interrogated to determine the presence and amount of label for each of the labeled binding partners.

Another aspect of the present invention relates to kits useful for conveniently performing the methods of the invention to analyze complex mixtures of proteins. To enhance the versatility of the subject invention, the reagents can be provided in packaged combination, in the same or separate containers, so that the ratio of the reagents provides for substantial optimization of the method and assay. The reagents may each be in separate containers or various reagents can be combined in one or more containers depending on the cross-reactivity and stability of the reagents.

A kit comprises in packaged combination a device in accordance with the present invention and labeled specific binding partners for each of the target proteins suspected of being present in a sample. The kit may also include washing solutions such as buffered aqueous medium for sample dilution as well as array washing, sample preparation reagents, and so forth. The relative amounts of the various reagents in the kits can be varied widely to provide for concentrations of the reagents that substantially optimize the reactions that need to occur during the present method and to further substantially optimize the sensitivity of the assay. Under appropriate circumstances one or more of the reagents in the kit can be provided as a dry powder, usually lyophilized, including excipients, which on dissolution will provide for a reagent solution having the appropriate concentrations for performing a method or assay in accordance with the present invention. The kit can further include a written description of a method in accordance with the present invention as described above.

It should be understood that the above description is intended to illustrate and not limit the scope of the invention. Other aspects, advantages and modifications within the scope of the invention will be apparent to those skilled in the art to which the invention pertains. The following examples are put forth so as to provide those of ordinary skill in the art with examples of how to make and use the method and products of the invention, and are not intended to limit the scope of what the inventors regard as their invention.

### Examples:

One example of a method in accordance with the present invention involves the analysis of cytokines in plasma. For purposes of example, the general preparation of simple arrays for assaying two cytokines, namely, TNF-alpha and Interleukin 6 (IL-6), is discussed. Of course, arrays composed of a larger number of features may also be employed. The following details can be varied in a number of ways to produce functional systems.

Phase 1: Array Preparation - For all of the aforementioned targets, the initial steps would be identical.
1. Identify a number of strong candidates for binding partners for each target, i.e., TNF-alpha and Interleukin 6 (IL-6). In a simple case, monoclonal antibodies are considered. Assuming a library of candidates has been prepared by conventional methods, each can be screened for binding affinity and specificity for each target, i.e., TNF-alpha and Interleukin 6 (IL-6). Based on these measurements, an initial primary capture agent is chosen.
2. The initial primary capture agent for each target protein, i.e., TNF-alpha and Interleukin 6 (IL-6), is immobilized onto a chromatographic support. A sufficient amount of the respective target protein is passed through the column under optimum binding conditions, saturating all the binding sites with target. The preselected second capture agent is then passed through the column. If it binds to an epitope different from the primary capture agent, it will be retained on the column. If it is not retained, then it can be concluded that it binds to the same epitope as the primary capture agent. In this case, the next candidate is evaluated. This procedure is repeated until three independent capture agents are determined. Two of these will be used for capture agents, while the third can be used as a reporter for the assay.
3. In this exemplary embodiment, the two capture agents are connected together with a triheterofunctional linker. Initially, the linker is immobilized onto the surface of a solid support. The second of three functionalities is chemically activated. The primary capture agent is passed over the support and attached to the linker. The remaining reactive site is then activated and reacted with the anchor site of the secondary capture agent. The linked capture agents are then released from the support and collected.
4. From this point, the procedure is conventional, similar to current approaches for production and use of protein or DNA arrays. The collected fractions are deposited into the array substrate surface in a spatially defined pattern using a deposition technique such as thermal or piezo inkjet deposition systems. The linked capture agents can either be adsorbed to the surface or covalently linked by the (now released) primary reactive site of the linker.

Phase 2: Sample Preparation - Sample preparation is also based on conventional technology. A typical sample is serum. Initially, the most abundant proteins are removed by affinity methods. For the molecular recognition process to function, it is important that the target proteins not be denatured. Therefore, non-denaturing conditions are maintained in terms salt, pH and organic content. In some cases, it might be necessary to dialyze the sample into the appropriate binding buffer. If detection is to be done indirectly, one may skip to Phase 3. If the samples are to be detected directly, the entire mixture is derivatized with fluorescent tag at a low level. It is desirable to optimize the binding to balance optimal detection sensitivity with minimal disruption of binding affinity. Typical fluorescent reagents such as NHS-Cy3 react non-specifically with amino groups and, thus, could potentially block binding epitopes.

Phase 3: Sample Application - Application of the sample to the array may be carried out by conventional techniques. The Array is carefully washed with an eluting buffer to reduce the background and then equilibrated with binding buffer. The sample is then applied and allowed to equilibrate with the surface. This is typically done by applying a volume of sample to the array and covering with a glass cover sheet and allowing the reagents to incubate. The excess sample proteins are then washed under mild conditions to remove any non-specific binding materials.

Phase 4: Detection - If the proteins are directly tagged with a fluorescent probe, then the array is ready to be imaged with a fluorescence scanner. For indirect detection, a fluorescently labeled third capture agent is added and allowed to bind to the captured targets and the resulting array is then imaged.

Phase 5: Data Processing - The data collected from the experiment described above can be processed in two distinct ways: relatively or absolutely. Relative quantitation generates information concerning expression levels for the target cytokines in a given sample relative to a control sample. If direct labeling of the target cytokine is used, then the above can be accomplished by using a conventional two color assay in which the two different samples (sample and control) are labeled with different dyes that exhibit spectrally differentiatable signals. A typical choice would be to use Cy3 NHS Ester and Cy 5 NHS Ester. If a sandwich assay is used with a second independent signaling affinity agent, then two color assays are not possible and two separate arrays must be used and compared.

Absolute quantitation can be achieved by using a standard set of arrays probed with a sample calibration set to generate a calibration curve. The signals obtained from the sample data can then be compared to the resulting calibration curve to generate absolute amounts of analyte present in the sample.

Either relative or absolute results can be further processed in a manner consistent with that used in the DNA array field.

Methods recited herein may be carried out in any order of the recited events, which is logically possible, as well as the recited order of events.

Although the foregoing invention has been described in some detail by way of illustration and example for purposes of clarity of understanding, it will be readily apparent to those of ordinary skill in the art in light of the teachings of this invention that certain changes and modifications may be made thereto

Furthermore, the foregoing description, for purposes of explanation, used specific nomenclature to provide a thorough understanding of the invention. However, it will be apparent to one skilled in the art that the specific details are not required in order to practice the invention. Thus, the foregoing descriptions of specific embodiments of the present invention are presented for purposes of illustration and description; they are not intended to be exhaustive or to limit the invention to the precise forms disclosed. Many modifications and variations are possible in view of the above teachings. The embodiments were chosen and described in order to explain the principles of the invention and its practical applications and to thereby enable others skilled in the art to utilize the invention.

The disclosures in United States patent application no. 10/405,438, from which this application claims priority, and in the abstract accompanying this application are incorporated herein by reference.

## Claims

1. A method for detecting one or more target proteins in a sample suspected of containing a plurality of said target proteins, said method comprising:
(a) incubating an assay medium comprising said sample with a surface of a substrate comprising a plurality of capture agents bound thereto in a predetermined pattern, wherein for each target protein at least two capture agents specific for different binding sites on said target protein are employed and wherein said two capture agents both bind to said target protein, said assay medium being incubated under conditions for binding of said capture agents to said target proteins, and
(b) examining said substrate surface for the presence of said target proteins.

2. A method according to claim 1 wherein each of said capture agents is a synthetic protein, natural protein, aptamer, synthetic peptide, natural peptide or synthetic affinity reagent.

3. A method according to claim 1 or 2 wherein each of said capture agents is independently an antibody or an antibody fragment.

4. A method according to any preceding claim wherein said examining of said substrate comprises contacting said substrate with a specific binding partner for each of said target proteins.

5. A method according to claim 4 wherein said specific binding partners for said target proteins a synthetic protein, natural protein, aptamer, synthetic peptide, natural peptide or synthetic affinity reagent.

6. A method according to claim 4 or 5 wherein said specific binding partners for said target proteins comprise a detectable moiety.

7. A method according to claim 6 wherein said detectable moiety is a luminescer, enzyme, enzyme substrate, radioactive group, particle, or promoter.

8. A method according to claims 4 to 7 wherein said specific binding partners are antibodies or antibody fragments.

9. A method according to any preceding claim wherein said examining of said substrate comprises contacting said substrate surface with a wash fluid and observing said substrate surface or said wash fluid for the presence of said target proteins.

10. A method according to any preceding claim wherein said substrate surface comprises an array of said capture agents.

11. A method according to claim 10 wherein said examining comprises observing a binding pattern on said array and evaluating the presence of the target proteins based on the observed binding pattern.

12. A device for determining one or more different target proteins (20, 22, 24) in a sample suspected of containing a plurality of said target proteins, said apparatus comprising:
(a) a solid substrate (11) comprising a surface (11a), and
(b) a plurality of capture agents (26a, 26b, 28a, 28b) bound to said surface in a predetermined pattern, wherein at least two capture agents specific for different binding sites on each of said target proteins are present and wherein said two capture agents are spatially disposed to both bind to a single molecule of said target protein.

13. A device according to claim 12 wherein each of said capture agents is a synthetic protein, natural protein, aptamer, synthetic peptide, natural peptide or synthetic affinity reagent.

14. A device according to claim 12 or 13 wherein said capture agents are present on said surface in an array.

15. A kit comprising in packaged combination a device according to any of claims 12 to 14 and a plurality of specific binding partners, one for each of said proteins.
